# EUROPEAN PATENT APPLICATION

(11) **EP 3 081 095 A1**
(43) Date of publication of application: **19.10.2016**
(21) Application number: 16164890.2
(22) Date of filing: 12.04.2016
(51) Int. Cl.: A23K 20/10, A23K 50/10, A23K 20/28, A23K 50/30

(54) **ANIMAL FEED COMPOSITIONS INCLUDING SPENT FILTER MEDIA CONTAINING DIATOMACEOUS EARTH**

(30) Priority: 13.04.2015 US 201562146708 P
(71) Applicant: Kent Nutrition Group, Inc., Muscatine, IA 52761 (US)
(72) Inventor: JOHAL, Sarjit, Muscatine, Iowa 52761 (US); SACHTLEBEN, Steve, Muscatine, Iowa 52761 (US); EDMONDS, Michael, Muscatine, Iowa 52761 (US); DENNIS, Rodney, Muscatine, Iowa 52761 (US)
(74) Representative: WSL Patentanwälte Partnerschaft mbB

(57) **Abstract**

Disclosed is an animal feed composition that includes spent filter media and animal nutritive components. The spent filter media may be present in any suitable amount, such as about 0.1 wt. % to about 5 wt. % by weight. The spent filter media includes spent diatomaceous earth, organic carbon, activated carbon and water. Methods of promoting weight gain of animals by administering the animal feed composition are also disclosed. In some embodiments, feeding a herd of animals with the animal feed composition will promote weight gain in swine relative to an otherwise-similar animal feed composition that does not include spent filter media.

## Description

### FIELD

The disclosure relates to animal feed compositions and associated methods of preparation and use.

### BACKGROUND

Animal growth promoters and enhancers are materials that facilitate the growth of animals, particularly, farm animals such as swine, livestock, poultry, and aquaculture, where the value of the herd of such animals to the farmer depends in part on herd weight. The market for animal growth promoters and enhancers is large and growing. The most widely used group of growth promoters in animal feed is antibiotics. Other growth promoters include probiotics, prebiotics, essential oils, botanicals, enzymes, organic acids, phytochemicals, vaccines, RNAs, antibodies, bacteriophage, antimicrobials, innate defense molecules, immune enhancers, and combinations thereof. There remains room for the development of new growth enhancers, particularly those that can be made in a cost-effective way.

An entirely separate set of issues arises from the use of vacuum filtration in the manufacture of certain agricultural, food, beverage, and biotechnology industries. In addition to the fixed equipment, production scale vacuum filtration systems such as rotary drum vacuum filters, leaf filters, and the like typically employ filtration aids. Such consumable aids range from fibers, cloths, and the like to particle materials such as clays, diatomaceous earth, carbon, cellulosics and associated materials and are used to coat the filters. For example, diatomaceous earth is a widely used filter medium. Diatomaceous earth is often used, alone or in conjunction with other process aids, to clarify water, beverages including beers, wines, juices and the like, and also to clarify food ingredients such as maltodextrins, syrups and the like. Such materials retain both insoluble and soluble impurities by various physicochemical means.

A problematic aspect of filtration systems that employ a filtration aid is the disposal of the used (spent) filtration aid (i.e., spent filter media). Disposal of the spent filtration media can be expensive. Because of the significant cost and time entailed in the recycling of filtration aids, the spent filter media is often disposed of in a landfill. However, disposing of this spent filter media in a landfill is wasteful and unproductive. Methods attempting to resolve this issue are known. For example, U.S. Patent No. 8,479,409 describes drying the spent filter media and using the dried spent filter media for treating soil.

Diatomaceous earth found in spent filter media (i.e., spent filter cake) is generally not considered as being suitable for use in animal feed applications due to the presence of expended or partially expended activated carbon. This material is thought to be undesirable in animal feed applications because of its high binding affinity. This high binding affinity generally creates an apprehension that the activated carbon will bind the nutrients in the animal feed and prevent the nutrients in the animal feed from being absorbed by the animals, which could lead to animal malnutrition.

### SUMMARY

It has surprisingly been found that when spent filter media can be incorporated into an animal feed composition without significantly reducing the nutritive value of the composition. The animal feed composition not only remains palatable to swine and cows, but also may promote weight gain in swine relative to an otherwise-similar animal feed composition that does not include spent filter media. Because in some embodiments the spent filter media can be supplied inexpensively to a swine or dairy farmer, the incremental increase in the value of the herd due to the increased weight gain can exceed the incremental cost of incorporating the spent filter media into the feed, thus resulting in positive cash flow for the farmer. An animal feed composition may comprise from about 0.1 wt. % to under 50%, such as up to about 5 wt. % spent filter media, the spent filter media including spent diatomaceous earth, organic carbon, activated carbon and water. The balance of the feed will comprise animal nutrients and optionally other feed-compatible materials such as fillers.

Also provided is method of promoting weight gain of animals, the method comprising feeding such animal feed composition to an animal. The animal may be any livestock or pet animal, for instance, swine, cow, or poultry. In some embodiments the feeding may be effective to promote weight gain in swine relative to an otherwise-similar animal feed composition that does not include spent filter media. Further provided is a method for preparing an animal feed. This method generally comprises blending the spent filter media with other feed components to form a mixture, and optionally forming the mixture into pellets.

### DETAILED DESCRIPTION

In various, non-mutually-exclusive embodiments, the invention provides animal growth-promoting feed compositions including spent filter media, methods of making such animal feed compositions, and methods administering such animal feed compositions to the animals. As discussed in more detail below, a feed made with spent filter media is palatable to swine and cows, and in some cases may enhance weight gain and may result in a desirable lower ratio of feed weight to weight gain.

The spent filter media containing diatomaceous earth can be obtained from a conventional commercial brewing process, but it will be appreciated that any suitable type of spent filter media that contains diatomaceous earth that is obtained from other processes may be incorporated into the animal feed compositions described herein. In various aspects, the spent filter media includes diatomaceous earth, organic carbon, activated carbon, and water, as well as organic materials derived from the brewing or other process. These may include biomolecules such as proteins, carbohydrates, fats, oils, vitamins, nucleic acids and low molecule weight metabolites, minerals including macro and micro minerals, as well as a range of other nourishing substances and material. As such, the spent filter media advantageously constitutes a concentrated form of feed extracts having nutritional and/or growth promoting properties when administered to animals as part of an animal feed composition.

In one form, an animal feed composition includes about 0.1 wt. % to about 50% spent filter media. The range may be otherwise characterized, and thus, for instance, the lower end of the range may be about 0.2%, 0.25%, 0.3%, 0.4%. 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%, and the upper end of the range may be, for instance, 45%, 40%, 35%, 30%, 25%, 20%, 15%,10%, 5%, 4%, 3%, 2%, or 1%. The range may be expressed as any permutation of the above, and thus, for instance, the range may be from about 0.1 % to about 5% by weight spent filter media; in some cases. 0.75%-3.5%, and in some cases 1-3%. The balance of the composition will include other animal feed components other than the spent filter media. Exemplary feed stocks are disclosed, for instance, in U.S. Patents 7,223,436 and 6,746,698, both of which are hereby incorporated by reference. For example, exemplary feedstocks may be made in accordance with the following formulations.

**Table 1: Animal Feed Formula 1**

| | |
|---|---|
| Ground Full-Fat Corn Germ | 36.8% |
| Wheat Midds | 33.1% |
| Ground Whole Corn | 17.9% |
| Soybean Meal | 9.5% |
| Corn Oil | 0.5% |
| Calcium Carbonate | 1.1% |
| Dicalcium Phosphate | 1.1 |

**Table 2: Animal Feed Formula 2**

| Ingredient | Weight % |
|---|---|
| Corn Germ | 35% |
| Wheat Midds | 31.5% |
| Whole Ground Corn | 17% |
| Soybean Meal | 9% |
| Hemicellulose | 5% |
| Calcium Carbonate | 1% |
| Dicalcium Phosphate | 1% |
| Corn Oil | 0.5% |

**Table 3: Animal Feed Formula 3**

| | |
|---|---|
| Full-Fat Corn Germ | 36% |
| Wheat Midds | 36% |
| Ground Corn | 15% |
| 48 Soybean Meal | 6.5% |
| Distillers Dried Grain | 2% |
| Calcium Carbonate | 1% |
| Dried Whole Wheat | 1% |
| Dehydrated Alfalfa | 1% |
| Dicalcium Phosphate | 1 % |
| Flavorized Vegetable Oil | 0.5% |

For instance, the animal feed composition may include a fat. It is contemplated that any animal and/or vegetable fat may be useful in conjunction with the invention. Suitable examples of vegetable fat include corn oil and soy oil. For instance, in one embodiment of the invention, full-fat corn germ is used as a starting material in fabricating the animal feed. An example of a suitable animal fat is choice white grease, a swine-derived fat. However, particularly in light of the growing concern over incorporation of animal by-products into animal feeds, preferred embodiments of the invention do not include animal fat.

When employed, the fat may be present in the animal feed in any amount effective to provide nutritive fat to the animal. It is contemplated that the fat content may vary depending upon the animal or upon the intended nutritive qualities of the feed. Generally, it is preferred that the fat is present in the animal feed in an amount of at least 5% by weight of the feed. More preferably, the fat is present in an amount of at least about 10%; even more preferably, an amount of at least about 15%; even more preferably, an amount of at least about 17%; even more preferably, an amount of at least about 20%; and even more preferably, an amount of at least about 25% by weight of the animal feed. It is contemplated that two or more fat sources may be included in the feed; if such is the case, the total fat amount preferably falls within one or more of the foregoing ranges.

The animal feed further includes a solid nutritive source. Any suitable solid nutritive source may be used in conjunction with the invention, and thus, for instance, the solid nutritive source may comprise a whole grain, such as whole wheat, whole rice, whole corn, or whole barley. The solid nutritive source alternatively may comprise a nutritive grain fraction, such as nutritive wheat, nutritive rice, nutritive corn, or nutritive barley fraction. Other nutritive sources include those derived from soy, oats, sorghum, and the like. The nutritive source may include other nutritive sources, including sources (such as molasses solids) that are initially provided in liquid form. The solid nutritive source may be present in the animal feed in any suitable amount. In the case of a somewhat low-fat feed, it is contemplated that the solid nutritive source may be present in an amount of 95% or greater. In more preferred embodiments of the invention, the solid nutritive source is present in an amount of at least about 60%, more preferably, an amount of at least about 70 % by weight of the animal feed.

It is preferred that the nutritive source include a protein source, which may be present in any amount effective to provide protein to the animal. Protein preferably is present in an amount ranging from about 5% to about 40% by weight of the animal feed. Young swine are particularly needy of protein, and protein contents in the upper portion of this range (e.g., a protein content of about 36%) are preferred in feeds intended for such swine. More preferably, for feeds for other animals, the protein is present in an amount ranging from about 10% to about 30% by weight of the animal feed; even more preferably, the protein is present in an amount ranging from about 15% to about 20% by weight of the animal feed.

It is further preferred that the animal feed include a fiber source. Generally, sources of fiber, such as soybean hulls, rice hulls, corn hulls, cottonseed, wheat hulls, and the like are considered largely non-nutritive (at least in the case of non-ruminant animals). In any case, regardless of whether the animal feed is intended for use by ruminants, the feed preferably includes such fiber source in an amount effective to provide fiber to the animal. Different feed formulas for different animals vary greatly in the amount of fiber desired. Preferably, the fiber source is prepared in an amount ranging from about 1% to about 25% by weight of the animal feed, the percentage being expressed by the bulk weight of the hulls or other source.

The feed may further include additional materials. For instance, the feed may include one or more vitamins or nutritive minerals, or, more generally, any other suitable nutritive source or other suitable ingredients. As but one example, the feed may include one or more antibiotics. More generally, the foregoing discussion is intended to provide guidelines as to the amount of ingredients suitable for use in the compositions of the invention. The actual composition of an animal feed may vary, depending on factors such as the type of animal and the desired levels of fat, fiber, nutrients, and other materials.

The animal feed compositions described herein may be prepared and tailored for, and advantageously administered to, a variety of animals, including but not limited to, ruminant and monogastric meat producing animals such as beef and dairy cattle, swine, sheep, lambs and goats, farm livestock including equine animals, poultry such as chickens, ducks, turkeys, and aquaculture farmed fish and shrimp for human consumption. Also contemplated are feed products for pet animals such as cats and dogs. When administering to swine, an exemplary feed suitable for use with the animal feed compositions described herein is one of the NexGen Program feeds available from Kent Nutrition Group, Inc. of Muscatine, Iowa. For calves, an exemplary feed suitable for use with the animal feed compositions described herein is HP Calf Creep 60B, available from Kent Nutrition Group, Inc. of Muscatine, Iowa.

The spent filter media described herein may be prepared using a variety of methods. For instance, the spent filter media may be dried using commercial drying systems and provided as a stand-alone product in dry or semi-dry powder or powder-like form, loose meal, pellets, granulated particles, extruded forms, or other suitable form. In such a case, the animal feed composition according to the principles described herein may be prepared (e.g., on-site by a farmer) simply by blending the spent filter media with a conventional animal feed to form a mixture. Alternatively, the spent filter media may be provided in the form of a final product including both the spent filter media and a suitable conventional animal feed such that the final product may be directly administered by the famers to the animals. In some cases, pellets of animal feed may be provided.

The spent filter media generally is sourced from a commercial filtration operation, such as a commercial food or food ingredient filtration or processing operation. Generally, the spent filter media will comprise 20-60% water by total weight, 20-70% diatomaceous earth by total weight, 15-30% organic carbon by total weight, this organic carbon largely constituting filtration solids inclusive of biomaterials, and 6-15% activated carbon by total weight. Spent filter media, as recovered from a rotary vacuum filter used in conventional commercial brewing processes, typically includes about 20-40 % water, 30-70 % diatomaceous earth, and 15-30% organic carbon by total weight of the spent filter media,, with activated carbon often not being present. Activated carbon is often used in the production of commercial maltodextrin and thus spent filter media from a maltodextrin production line may have 5-15% activated carbon. These are general ranges and a particular filtration process may provide spent filter media with these components present in other amounts.

It will be appreciated that the spent filter media as described herein can be fed to the animals in its wet form (e.g., as recovered following a conventional brewing process from the rotary drum vacuum filter). Preferably, though, spent filter media having high levels of moisture may be dried (e.g., via sterilization, another heat treatment, chemically treated such as with hydrogen peroxide or other agents, or another suitable treatment) to reduce the potential for bacterial contamination.

In one approach, the spent filter media can be dried using, for example, a steam jacket ribbon blender. The spent filter media can be dried to a moisture content of about 15% or less in one approach, about 10% or less in another approach, and about 5% or less in yet another approach and about 1% or less in still another approach. It will be appreciated that the spent filter media may also be used in the animal feed compositions described herein without being pre-dried. By one approach, instead of being dried with a device such as a steam jacket ribbon blender, the spent filter media may be blended with one or more dry materials to form a spent filter media composition as described, for example, in U.S. Patent No. 8,479,409, incorporated by reference in its entirety herein.

When this approach is employed, dry materials suitable for being blended with the spent filter media include, but are not limited to, organic materials such as corn bran or distillers' dried grains, or the like. One suitable dry material is SOLULAC®, sold by Grain Processing Corporation of Muscatine, Iowa. SOLULAC® comprises corn fiber augmented with distillers' grain solubles. A dried spent filter media composition may include about 2 wt. % to about 30 wt. % of a nutrient such as SOLULAC® based on the total weight of the spent filter media composition.

Prior to blending with the spent filter media, the dry material need not be completely moisture-free, and in some embodiments, the dry material includes some moisture, but is sufficiently low in moisture to dry the spent filtration media to the requisite level of dryness (e.g., below 10 wt. %). The dry material added to the spent filter media may include less than 2 wt. % moisture in one approach and less than 1 wt. % moisture in another approach.

The dry material and spent filter media may be blended in any suitable fashion and in any ratio effective to dry the spent filter media to the desired moisture content, which generally is an amount (e.g., about 15% or less in one approach, about 10% or less in another approach, and about 5% or less in yet another approach and about 1% or less in still another approach) that will not support restrict and/or inhibit microbial growth. The dry material and spent filter media, along with any other desired optional ingredients, may be blended to produce a spent filter media composition containing 5 wt. % to 90 wt. %, preferably 40 wt. % to 70 wt. %, more preferably 40 wt. % to 60 wt. %, spent filter media. In some aspects, the dry material may be optionally present in amounts of about 15 wt. % and 60 wt. % based on the total weight of the spent filter media composition.

An animal feed composition prepared in accordance with the present teaching may be administered to animals in varying amounts (e.g., 0.25 pounds per day to 10 pounds per day, or 1-9 pounds per day, or 2-8 pounds per day) as appropriate for a particular animal to advantageously increase both the feed intake by the animals and the weight gain by the animals as compared to conventional animal feeds that do not include the spent filter media containing diatomaceous earth.

It is contemplated that the composition will be roughly as palatable to the intended animal as an otherwise-identical feed composition prepared in the absence of the spent filter media. In some embodiments, the composition may be effective to promote weight gain in an animal as compared to an otherwise similar feed without the spent filter media. One way to determine whether the feed is effective in this regard is to determine the average 21-day post-weaned animal weight gain as compared to an otherwise identical animal feed composition not including the spent filter media. To determine the average 21-day post-weaned weight gain, a herd of at least 25 of the animal in question (swine, poultry, etc.) is provided at weaning. The herd is fed to satiety over a period of 21 days beginning at weaning with the feed composition under evaluation. The average weight gain of each herd is then determined. Actual weight gain and feed efficiency are believed to be affected by numerous factors, including immune state, genetics, shipping and handling stresses, and environmental factors such as weather, housing, crowding and other associated factors.

Advantages and embodiments of the animal feed compositions described herein are further illustrated by the following non-limiting examples.

### Example 1 - Preparation of Spent Filter Media

A spent filter material (i.e., spent filter media or spent filter cake) was recovered from a rotary vacuum filter. The spent filter material contained about 35% moisture 32 % diatomaceous earth 22% organic carbon and 11% activated carbon. The spent filter material was dried using a steam jacket ribbon blender to a final moisture content of less than 10% and was cooled and packaged in 50 pound bags or drums.

### Example 2 - Swine Palatability

The dried spent filter material was added to a premium swine feed. Animal Feed 1 (Control) fed to Group 1 contained no dried spent filter media and 100% of the premium swine feed. Animal Feed 2 fed to Group 2 contained 5 pounds per ton (0.25 wt. %) of the spent filter media. Animal Feed 3 fed to Group 3 contained 20 pounds per ton (1 wt. %) of the spent filter media. Animal Feed 4 fed to Group 4 contained 40 pounds per ton (2 wt. %) of the spent filter media. Animal Feed 5 fed to Group 5 contained 80 pounds per ton (4 wt. %) of the spent filter media. The effect of the addition of various levels of the spent filter material to the premium swine feed is shown below in Table 4. In this and the following Tables, "SFC" refers to spent filter media (cake).

**Table 4:**

| Treatments | Group 1 (Feed 1 - Control) | Group 2 (Feed 2) | Group 3 (Feed 3) | Group 4 (Feed 4) | Group 5 (Feed 5) | Group 2 v. Group 1 | Group 3 v. Group 1 | Group 4 v. Group 1 | Group 5 v. Group 1 |
|---|---|---|---|---|---|---|---|---|---|
| SFC, lb./ton | 0 | 5 | 20 | 40 | 80 | | | | |
| # Pigs Tested¹ | 81(1) | 81 | 81 | 81 | 81(1) | | | | |
| # Pigs per Pen | 9 | 9 | 9 | 9 | 9 | | | | |
| Initial Weight (lbs.) | 14.2 | 14.2 | 14.2 | 14.5 | 14.4 | | | | |
| *Days 0-11* | | | | | | | | | |
| Average Daily Gain (lbs.) | .39 | .40 | .39 | .40 | .43 | 2.56% | 0% | 2.56% | 10.26% |
| Avg Daily Feed Intake (lbs.) | .46 | .45 | .46 | .47 | .47 | -2.17% | 0% | 2.17% | 2.17% |
| Ratio Feed/Gain | 1.20 | 1.13 | 1.19 | 1.18 | 1.11 | -5.83% | -0.83% | -1.67% | -7.50% |
| *Days 0-34* | | | | | | | | | |
| Average Daily Gain (lbs.) | .99 | .97 | .98 | .99 | .96 | -2.02% | -1.01% | 0% | -3.03% |
| Avg Daily Feed Intake (lbs.) | 1.46 | 1.45 | 1.47 | 1.52 | 1.41 | -0.68% | 0.68% | 4.11% | -3.42% |
| Ratio Feed/Gain | 1.48 | 1.50 | 1.49 | 1.53 | 1.46 | 1.35% | 0.68% | 3.38% | -1.35% |

One pig died (9.5 lb., Naval Prolapse) in Group 1 and one pig died (9.6 lb.) in Group 5.

In the Group 5 feed, additional nutrients and fat were added to the feed relative to the other groups. The addition of the spent filter media from 5 pounds per to 80 pounds per ton of the premium swine feed (which included prebiotics) was observed to have no adverse effects on animal health or performance during the first 11 days post-weaning. This Example demonstrates that the spent feed additive was palatable to the herd and was generally safe.

### Example 3 - Swine feed

Spent filter media was incorporated into a commercial, non-premium animal feed composition and administered to animals as follows.

**Table 5:**

| Treatments | Group 6 (Control) | Group 7 | Group 8 | Group 7 v. Group 6 | Group 8 v. Group 6 |
|---|---|---|---|---|---|
| SFC added, lb./ton | 0 | 5 | 20 | | |
| Added Animal Fat, lb./ton | | | 15 | | |
| # Pigs Tested (total) | 30(1)* | 30 | 30(1)* | | |
| # Pens | 6 | 6 | 6 | | |
| Initial Weight, lbs. | 44.69 | 45.03 | 45.32 | | |
| *Days 0-21* | | | | | |
| Avg Daily Gain, lbs. | 1.721 | 1.776 | 1.746 | 3.20% | 1.45% |
| Avg Daily Feed intake, lb. | 3.590 | 3.548 | 3.493 | -1.17% | -2.70% |
| Ratio Feed/Gain^{1,2} | 2.086 | 1.998 | 2.003 | -4.22% | -3.98% |
| *Days 0-112* | | | | | |
| Avg Daily Gain^{2,4}, lb. | 2.318 | 2.382 | 2.447 | 2.76% | 5.57% |
| Avg Daily Feed Intake, lb. | 6.446 | 6.479 | 6.502 | 0.51% | 0.87% |
| Ratio Feed/Gain³ | 2.783 | 2.720 | 2.659 | -2.26% | -4.46% |
| *Carcass* | | | | | |
| Percent Lean | 56.15 | 55.80 | 56.57 | | |

| | | | | | |
|---|---|---|---|---|---|
| *Group 6 had one dead pig (47 lb.); Group 8 had one dead pig (215 lb.). ¹6 vs 7 (P≤.05); ²6 vs 8 (P≤.05); ³6 vs 8 (P < .10); ⁴6 vs 7 (P = .15) | | | | | |

The data tended to suggest that feed efficiency was improved during days 0-21 when the pigs were fed the feed with spent filter media compared to the control animals. Also, during this time, the ratio of the feed consumed per weight gained was desirably reduced and the overall, (Days 0-112) weight gain was improved. Further, it was observed that the lean mass percentage of the animals remained substantially unchanged when using spent filter media.

### Example 4 - Swine feed (Grow-Finish Pigs)

Spent filter media was incorporated into a commercial, non-premium animal feed composition and administered to animals as follows.

**Table 6:**

| Treatments | Group 9 Control | Group 10 Test Group | Group 10 Test Group v. Group 9 Control |
|---|---|---|---|
| SFC added, lb./ton | 0 | 5 | |
| # Pigs Tested (total) | 80 | 80 | |
| # Pens | 9 | 9 | |
| Initial Weight, lbs. | approx. 41 lbs. | approx. 41 lbs. | |
| *Days 0-110* | | | |
| Avg Daily Gain, lbs. | 1.954 | 1.960 | 0.31% |
| Avg Daily Feed intake, lb. | 5.133 | 5.127 | -0.12% |
| Ratio Feed/Gain | 2.629 | 2.616 | -0.49% |
| *Deaths* | 4 | 4 | |

The data tended to suggest that animals in the test group demonstrated a better numerical response for average daily weight gain and feed efficiency as compared to the control. The 5% death rate was identical for both the control and test groups.

### Example 5 - Swine feed (Grow-Finish Pigs)

Wet or "as is" spent filter media from a commercial filtration process containing 35-40% moisture was incorporated into a commercial, non-premium animal feed composition and administered to animals as follows.

**Table 7:**

| Treatments | Group 11 Control | Group 12 | Group 13 | Group 12 v. Group 11 Control | Group 13 v. Group 11 Control |
|---|---|---|---|---|---|
| SFC added, lb./ ton | 0 | 7* | 14* | | |
| # Pigs Tested (total) | 42 | 42 | 42 | | |
| # Pens | 16 | 16 | 16 | | |
| Initial Weight, lbs. | approx. 43 lbs. | approx. 43 lbs. | approx. 43 lbs. | | |
| *Days 0-110* | | | | | |
| Avg Daily Gain, lbs. | 2.039 | 2.068 | 2.037 | 1.42% | -0.10% |
| Avg Daily Feed intake, lb. | 5.496 | 5.507 | 5.505 | 0.20% | 0.16% |
| Ratio Feed/Gain | 2.696 | 2.662 | 2.702 | -1.26% | 0.22% |
| *Deaths* | 2 | 4 | 1 | | |

| | | | | | |
|---|---|---|---|---|---|
| *SFC was used "as is" in this Example, and contained 35-40% moisture | | | | | |

The animals in Test Group 12 demonstrated an increase in the Average Daily Gain (ADG), Average Daily Feed Intake (ADFI), and feed efficiency (Feed/Gain) as compared to the control. The animals in Test Group 13 demonstrated a decrease in the ADG and feed efficiency, while also demonstrating an increase in the ADFI as compared to the control. ADG and feed efficiency were better as compared to the control with Test Group 1 than Test Group 13, while ADFI was slightly better as compared to the control in Test Group 13 than Test Group 12.

### Example 6 - Bovine feed

Seventy multiparous beef cows and their calves were separated into two pastures. The cows in Group 14 were fed HP Calf Creep 60B with no spent filter media added, while the cows in Group 15 were fed HP Calf Creep 60B with 5 wt. % spent filter media added. The feeds were available to the animals by choice throughout the entirety of the study. Feed intake was calculated as the difference between feed offered minus feed left at the termination of the study 38 days later. The impact of feeding the spent filter media on the feed intake by the calves is summarized in Table 3 below.

**Table 8:**

| Treatment | Group 14 | Group 15 |
|---|---|---|
| Description | Control Creep Feed | Control Creep Feed with Spent Filter Media |
| Feed Intake Pounds/day | 3.65 | 4.46 |
| % Growth Improvement | | +22.2% |

The data tended to show that calves assigned to animal feed including the spent filter media (Treatment 2) consumed more feed (4.46lb/head/day) compared to those animals that were fed the animal feed not including the spent filter media (3.65 lb./head/day). The use of 5% spent filter media in the animal feed thus resulted in an enhanced feed intake by the animals.

### Example 7 - Bovine feed (Holstein Calves)

Two four-week feeding tests were conducted with spent filter media to determine acceptance by growing Holstein steers beginning at approximately 240 pounds body weight. In both tests a commercial type pelleted calf grower containing 16% protein was used as the basal ration. The Control feed did not contain any added SFC and the test feed fed to Test Group 17 and Test Group 19 contained 100 pounds/ton (5% inclusion) of SFC. In the test feed the amount of wheat middlings and soybean oil meal were adjusted to incorporate the 100 lbs. of SFC. Both feeds were of similar nutrient content. Feeds were offered *ad libitum* in self feeders.

**Table 9:**

| Treatments | Group 16 Control¹ | Group 17 Test Group | Group 17 Test Group v. Group 16 Control |
|---|---|---|---|
| No. Calves | 24 | 23 | |
| Start Wt. lbs. | 240.42 | 242.65 | |
| End Wt. lbs. | 321.83 | 321.00 | |
| Gains lbs. | | | |
| Total Gains lbs. | 81.41 | 78.35 | -3.76% |
| Avg Daily Gain, lbs. | 3.02 | 2.90 | -3.97% |
| Feed Intake | | | |
| Total Feed Intake lbs. | 274.20 | 251.90 | -8.13% |
| Avg Daily Feed intake, lbs. | 9.79 | 9.00 | -8.07% |
| Ratio Feed/Gain | 2.696 | 2.662 | -1.26% |

| | | | |
|---|---|---|---|
| ¹The Control feed was a 16% protein commercial calf grower; ²The Test Group feed was a 16% protein commercial calf grower containing 5% SFC. | | | |

**Table 10:**

| Treatments | Group 18 Control¹ | Group 19 Test Group | Group 19 Test Group v. Group 18 Control |
|---|---|---|---|
| No. Calves | 24 | 23 | |
| Start Wt. lbs. | 356.25 | 355.17 | |
| End Wt. lbs. | 453.21 | 458.39 | |
| Gains lbs. | | | |
| Total Gains lbs. | 96.96 | 103.22 | 6.46% |
| Avg Daily Gain, lbs. | 3.34 | 3.56 | 6.59% |
| Feed Intake | | | |
| Total Feed Intake lbs. | 425.00 | 437.00 | 2.82% |
| Avg Daily Feed intake, lbs. | 14.66 | 15.07 | 2.80% |
| Ratio Feed/Gain | 4.38 | 4.23 | -3.42% |

| | | | |
|---|---|---|---|
| ¹The Control feed was a 16% protein commercial calf grower; ²The Test Group feed was a 16% protein commercial calf grower containing 5% SFC; | | | |

In Test Group 17, steers gained an average of 3.07 pounds less body weight; had 0.1 pound less average daily gain (ADG); and ate less feed. The most significant result was improved feed efficiency (Feed/Gain) in that steers in Test Group 17 required 0.15 pound less feed per pound of gain. In Test Group 19, steers gained an average of 6.26 pounds more body weight; had 0.22 pound more average daily gain (ADG); and ate slightly more feed. Notably, steers in Test Group 19 required 0.15 pound less feed per pound of gain.

The data established generally that the feed was palatable to the steers. The data further suggested that 5% of the spent filter media had a positive effect on feed efficiency.

The results of both tests demonstrated that 5% SFC in the diet had a positive effect on feed efficiency and did not significantly affect feed intake, performance, or apparent health of steers.

### Example 8 - Bovine feed (Holstein Steer)

The Test diets for the trial were formulated to contain 4% spent filter media on an as-fed basis, 35% wet gluten, 22.5 % ground hay and the balance corn and balancer. The Control diet was the same with the exception of the SFC and that amount was added to the hay concentration. Diets were isonitrogenous and isocaloric.

**Table 11:**

| Treatments | Group 20 Control | Group 21 Test | | Group 21 Test v. Group 20 Control |
|---|---|---|---|---|
| *Days* (*0-28*) | | | | |
| Avg Daily Gain, lbs. | 1.93 | 2.49 | | 29.02% |
| Avg Daily Feed intake, lbs. | 15.65 | 17.25 | | 10.22% |
| F/G | 8.16 | 6.96 | | -14.71% |
| *Days (29-56)* | | | | |
| Avg Daily Gain, lbs. | 3.53 | 3.15 | | -10.76% |
| Avg Daily Feed intake, lbs. | 19.71 | 21.05 | | 6.80% |
| F/G | 5.65 | 6.85 | | 21.24% |
| *Days (0-56)* | | | Difference (lbs.) over 56 days | |
| Avg Daily Gain, lbs. | 2.67 | 2.79 | 6.72 | 4.49% |
| Avg Daily Feed intake, lbs. | 18.52 | 19.7 | 66.08 | 6.37% |
| Ratio Feed/Gain | 6.91 | 7.04 | | 1.88% |

**Table 12:**

| *Days* (*55-81*) | Group 22 Control¹ | Group 23 Test | Group 23 v. Group 22 Control |
|---|---|---|---|
| Avg Daily Gain, lbs. | 1.93 | 2.49 | 29.02% |
| Avg Daily Feed intake, lbs. | 15.65 | 17.25 | 10.22% |
| Ratio Feed/Gain | 8.16 | 6.96 | -14.71% |

The data in Table 11 is from two replicates per treatment. The second replicate was also carried a third weigh period and those data (55-81 days) are in Table 12. Extra body weight gained over 56 days was about 6.72 pounds. The test cattle also ate about 66.08 pounds more during that period. Nonetheless, the feed efficiency (Feed/Gain), was comparable over the 56 days. The young TRT animals, (0-28 days) and the older TRT animals (55-81 days) fed the Test diet exhibited better feed efficiency than the controls.

### Example 9 -- Swine feed (Grow-Finish Pigs)

One hundred fifty-eight (158) pigs of mixed gender were evaluated in a 110-day feeding study. The treatment design consisted of 12 replicates per treatment with 12-13 pigs per pen. The initial body weights of the nursery pigs were approximately 50 lbs. each, and the final recorded body weight were about 269 lbs. each.

The study protocol specified two treatment levels 5 lbs. and 10 lbs. of dry spent filter media. However, after about 60 days the availability of dried SFC was exhausted due to drying equipment issues. Consequently, for the remaining 50 days of the trial, undried "as is" spent filter media was substituted and inclusion rates for the treatment groups were adjusted to account for the added moisture to 7 lbs. and 14 lbs. respectively.

The following results were obtained.

**Table 13:**

| | Group 24 Control | Group 25 | Group 26 | Group 25 vs. Group 24 Control | Group 26 vs. Group 24 Control |
|---|---|---|---|---|---|
| | | 5 or7 lbs.* | 10 or 14 lbs.* | | |
| Avg Daily Gain, lbs. | 1.963 | 1.995 | 1.976 | 1.63% | 0.66% |
| Avg Daily Feed intake, lbs. | 5.269 | 5.422 | 5.324 | 2.90% | 1.04% |
| Ratio Feed/Gain | 2.683 | 2.720 | 2.694 | +1.38% | +0.41% |
| Deaths | 1 | 1 | 1 | | |

| | | | | | |
|---|---|---|---|---|---|
| *SFC was used 'as is' and contained 35-40% moisture. | | | | | |

As in several of the earlier examples, both Groups 25 and 26 exhibited positive average daily gain and average daily feed intake relative to the control, but feed efficiency was less.

In the above Examples 3, 4, 5, and 9, a total of 474 pigs in 52 pens were evaluated at the 5 lb. (or 7 lb. "as is") spent filter media usage rate versus controls, and a total of 284 animals in 28 pens were evaluated at the 10 lb. (or 14 lb. wet) usage rate versus controls. The average daily weight gain, average daily feed intake, and feed efficiency values were calculated as average values, without accounting for other variables in the study. The following gross aggregate average values were determined.

**Table 14:**

| | 5 lb. (or 7 lb.) vs control | 10 lb. (or 14 lb.) vs control |
|---|---|---|
| Avg Daily Gain, lbs. | 1.24% | 0.23% |
| Avg Daily Feed intake, lbs. | 0.75% | 0.54% |
| Ratio Feed/Gain | -0.50% | 0.30% |

While not definitive, this aggregated data suggests that the spent filter media improves average daily weight gain and feed intake, and may improve the feed efficiency.

The animal feed compositions as described herein may be fed to different animals. It is believed that the use of spent filter media may advantageously increase the growth of the animals on a daily basis as compared to identical amounts of conventional animal feed compositions, although this result is believed to be affected by other conditions including the composition of the base feed. In addition, the animal feed compositions as described herein have been observed in the above Examples not to adversely affect the lean mass percentage of the animals and not to exhibit any detrimental effects to the growth or the health of the animals.

The foregoing descriptions are not intended to represent the only forms of the animal feed compositions in regard to the details of the formulation. Changes in form and in proportion of parts, as well as the substitution of equivalents, are contemplated as circumstances may suggest or render expedient. More generally, all methods described herein can be performed in any suitable order unless otherwise indicated herein or otherwise clearly contradicted by context. The use of any and all examples, or exemplary language (e.g., "such as") provided herein, is intended to illuminate the invention and does not pose a limitation on the scope of the invention unless otherwise claimed. No language in the specification should be construed as indicating any non-claimed element as essential to the practice of the invention. This invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An animal feed composition comprising animal feed components including animal nutrients and about 0.1 wt. % to about 5 wt. % spent filter media, the spent filter media including spent diatomaceous earth, organic carbon, activated carbon and water.

2. The animal feed composition of claim 1, wherein the spent filter media is a dried material including about 0 wt. % to about 10 wt. % water.

3. The animal feed composition of claim 1, wherein the spent filter media is present in the animal feed composition in an amount of about 0.75 wt. % to about 3 wt. %.

4. The animal feed composition of claim 1, including a fiber source present in an amount ranging from about 1% to about 25% by weight of the animal feed.

5. The animal feed composition of claim 1, including a fat source present in an amount of at least about 15%.

6. The animal feed composition of claim 1, including a protein source in an amount ranging from about 10% to about 30% by weight of the animal feed.

7. A method of feeding an animal, the method comprising:
providing an animal feed composition comprising animal feed components including animal nutrients and about 0.1 wt. % to about 5 wt. % spent filter media, the spent filter media including spent diatomaceous earth, organic carbon, activated carbon and water., and
feeding said animal feed composition to an animal.

8. The method of claim 7, wherein the spent filter media is a dried material including about 0 wt. % to about 10 wt. % water.

9. The method of claim 7, wherein the spent filter media is present in the animal feed composition in an amount of about 0.75 wt. % to about 3 wt. %.

10. The method of claim 7, where the animal feed composition includes a fiber source present in an amount ranging from about 1% to about 25% by weight of the animal feed.

11. The method of claim 7, where the animal feed composition includes a fat source present in an amount of at least about 15%.

12. The method of claim 7, where the animal feed composition includes a protein source in an amount ranging from about 10% to about 30% by weight of the animal feed.

13. The method of claim 7, including blending the spent filter media with the other components of the animal feed composition.
